# NEUE EUROPÄISCHE PATENTSCHRIFT

(11) **EP 3 183 013 B2**
(45) Veröffentlichungstag und Bekanntmachung des Hinweises auf die Entscheidung über den Einspruch: **07.10.2026**
(45) Hinweis auf die Patenterteilung: 09.10.2019
(21) Anmeldenummer: 15754118.6
(22) Anmeldetag: 17.08.2015
(51) Int. Cl.: A61M 1/16, A61M 1/34

(54) **DIALYSEMASCHINE MIT DER FÄHIGKEIT ZUR BESTIMMUNG EINER PRÄDIALYTISCHEN EIGENSCHAFT IM BLUT EINES DIALYSEPATIENTEN**
DIALYSIS MACHINE WITH THE CAPABILITY OF DETERMINING A PRE-DIALYSIS PROPERTY IN THE BLOOD OF A DIALYSIS PATIENT
MACHINE DE DIALYSE AYANT LA CAPACITÉ DE DÉTERMINER UNE PROPRIÉTÉ PRÉ-DIALYTIQUE DANS LE SANG D'UN PATIENT SOUS DIALYSE

(30) Priorität: 20.08.2014 DE 102014012423
(43) Veröffentlichungstag der Anmeldung: 28.06.2017
(73) Patentinhaber: Fresenius Medical Care Deutschland GmbH, 61352 Bad Homburg (DE)
(72) Erfinder: MAIERHOFER, Andreas, 97422 Schweinfurt (DE)
(74) Vertreter: Lorenz Seidler Gossel Part. mbB
(86) Internationale Anmeldenummer: PCT/EP2015/001690
(87) Internationale Veröffentlichungsnummer: WO 2016/026569

(56) Entgegenhaltungen:
- EP-A1- 0 291 421
- WO-A1-2010/112223
- WO-A1-2010/112223
- GOUREAU Y ET AL: "EVALUATION OF PLASMA SODIUM CONCENTRATION DURING HEMODIALYSIS BY COMPUTERIZATION OF DIALYSATE CONDUCTIVITY", ASAIO TRANSACTIONS, HARPER AND ROW PUBLISHERS, HAGERSTOWN, MD, US, vol. 36, no. 3, 1 July 1990 (1990-07-01), pages 444 - 447, XP000204537

## Beschreibung

Die Erfindung betrifft eine Dialysemaschine mit einem extrakorporalen Blutkreislauf, einem Dialysat-Kreislauf, einem Dialysator und einer Recheneinheit, wobei im Dialysatkreislauf wenigstens ein Sensor zur Bestimmung einer Eigenschaft des Dialysats angeordnet ist. Die Recheneinheit hat die Fähigkeit zur Abschätzung einer prädialytischen Eigenschaft im Blut eines Dialysepatienten.

Die Natriumionenkonzentration im Blutplasma eines Dialysepatienten ist ein wichtiger diagnostischer Parameter, der den Arzt zu weitergehenden Untersuchungen sowie zu Anpassungen sowohl des Dialyseregimes als auch der medikamentösen Therapie (z.B. mit Diuretika, zur Glykämie-Kontrolle, etc.) veranlassen kann. Zudem korrelieren Mortalität und Morbidität mit der Variabilität der prädialytischen Natriumionenkonzentration im Blutplasma. Blutanalysen zur Bestimmung der Natriumionenkonzentration im Blutplasma sind aufwendig und kostspielig und werden daher kaum hinreichend häufig durchgeführt. Goureau et al. ("Evaluation of plasma sodium concentration during hemodialysis by computerization of dialysate conductivity", ASAIO Transactions, Bd. 36, Nr. 3, Jui 1990) offenbart eine Dialysemaschine mit zwei Leitfähigkeitssensoren im Dialysatkreislauf zur Bestimmung einer Natriumionenkonzentration im Blutplasma des Dialysepatienten.

EP0291421 A1 offenbart eine Dialysemaschine mit zwei Leitfähigkeitssensoren im Dialysatkreislauf und einer Recheneinheit, die derart ausgebildet ist, dass ermittelte Leitfähigkeitsmesswerte stromaufwärts des Dialysators zu einem ersten Zeitpunkt und stromabwärts des Dialysators zu einem späteren Zeitpunkt als Wertepaare zur Bestimmung der Natriumionenkonzentration des Patientenblutes herangezogen werden.

Im Stand der Technik sind Approximationen der prädialytischen Natriumionenkonzentration im Blutplasma aus Leitfähigkeitsmessungen im Dialysat während der Dialyse bekannt, die zwar ohne zusätzlichen Aufwand möglich sind, die aber ungenau sind und nur eine Näherung für einen Zeitpunkt angeben, zu dem sich die Natriumionenkonzentration im Blutplasma unter dem Einfluss der Dialyse schon wesentlich geändert hat.

Diese Methoden beruhen darauf, dass bei einer Dialysebehandlung die Elektrolyte im Blut mit denjenigen im Dialysat über den Kontakt im Dialysator im Gleichgewicht stehen. Aufgrund der Massenerhaltung ist es bei Kenntnis der Stoffkonzentrationen ĉ_{di} und ĉ_{do} auf der Dialysatseite stromaufwärts und stromabwärts des Dialysators, des Dialysatflusses durch den Dialysator Q_{d}, des Substituatflusses Qₛ, der UF-Rate Q_{f} und der Clearance K möglich, die Stoffkonzentration ĉ_{bi} auf der Bluteingangsseite zu berechnen. In M. Gross et al., "Online clearance measurement in high-efficiency hemodiafiltration", Kidney International, 72, p.1550 ff. wird eine allgemeine Formel zur Massenbilanz bei HD- und HDF-Behandlungen angegeben: ${{c}^{˜}}_{do} \left(t\right) = \left(1-\frac{K}{{Q}_{d} + {Q}_{s} + {Q}_{f}}\right) {{c}^{˜}}_{di} \left(t\right) + \frac{K {{c}^{˜}}_{bi} - \frac{d}{dt} M \left(t\right)}{{Q}_{d} + {Q}_{s} + {Q}_{f}}$ [1]

Umformung nach c_{bi} ergibt im stationären Zustand d/dt M(t)=0: ${{c}^{˜}}_{bi} = {{c}^{˜}}_{di} + \frac{{Q}_{d} + {Q}_{s} + {Q}_{f}}{K} \left({{c}^{˜}}_{do}-{{c}^{˜}}_{di}\right)$ [2]

Die Konzentration von Natriumionen in der Dialysierflüssigkeit ĉ_{d}^{Na} korreliert stark mit der temperaturkompensierten Leitfähigkeit c_{d} der Dialysierflüssigkeit.

In Dialysegeräten mit der Möglichkeit einer leitfähigkeitsbasierten Clearance-Messung kann die Leitfähigkeit des Dialysats stromaufwärts und stromabwärts des Dialysators kontinuierlich gemessen werden. Hierbei kann zugleich zu äquidistanten Zeitpunkten tᵢ eine Speicherung der Leitfähigkeitswerte c_{di}(tᵢ) und c_{do}(tᵢ) in einer Speichereinheit erfolgen. Dialysegeräte mit der Fähigkeit einer leitfähigkeitsbasierten Clearance-Messung werden beispielsweise in der EP 2 413 991 A1 oder der EP 1 108 438 B1 offenbart. Ferner gibt es Dialysegeräte, welche die Fähigkeit haben, zu den Zeitpunkten tᵢ den Dialysatfluss Q_{d}(tᵢ), den Blutfluss Q_{b}(tᵢ), den Substituatfluss Qₛ(tᵢ) und die Ultrafiltrationsrate Q_{f}(tᵢ) zu speichern, und so in weiterer Folge die Clearance K(tᵢ) zu jedem Zeitpunkt tᵢ zu bestimmen. Ein derartiges Dialysegerät wird beispielsweise in der EP 1 062 960 B1 offenbart. Damit kann dann die Äquivalent-Leitfähigkeit c_{bi}(tᵢ) am Bluteingang zum Zeitpunkt tᵢ berechnet werden: ${c}_{bi} \left({t}_{i}\right) = {c}_{di} \left({t}_{i}\right) + \frac{{Q}_{d} \left({t}_{i}\right) + {Q}_{s} \left({t}_{i}\right) + {Q}_{f} \left({t}_{i}\right)}{K \left({t}_{i}\right)} \left({c}_{do}\left({t}_{i}\right)-{c}_{di}\left({t}_{i}\right)\right)$ [3]

In der Praxis ist die Bestimmung von c_{bi}(tᵢ) aber aus diversen Gründen nicht mit einer sinnvollen Genauigkeit möglich. Denn zum einen besteht erst nach Konnektion des Patienten über den Dialysator ein Kontakt zwischen Blut und Dialysat, so dass ein Zusammenhang zwischen c_{do} und c_{bi} besteht. Unmittelbar nach Beginn der Dialyse ist das berechnete c_{bi} also entweder gleich C_{di} (falls in der Vorbereitungsphase eine Äquilibrierung zwischen Dialysierflüssigkeit und blutseitiger Spüllösung stattgefunden hat) oder enthält einen zufälligen Wert aus der Zeit, in der sich nach dem Konnektieren des Patienten Blut und Spüllösung solange vermischen, bis ein an der blutseitigen Rückgabeleitung befindlicher Detektor das Vorhandensein von Blut detektiert.

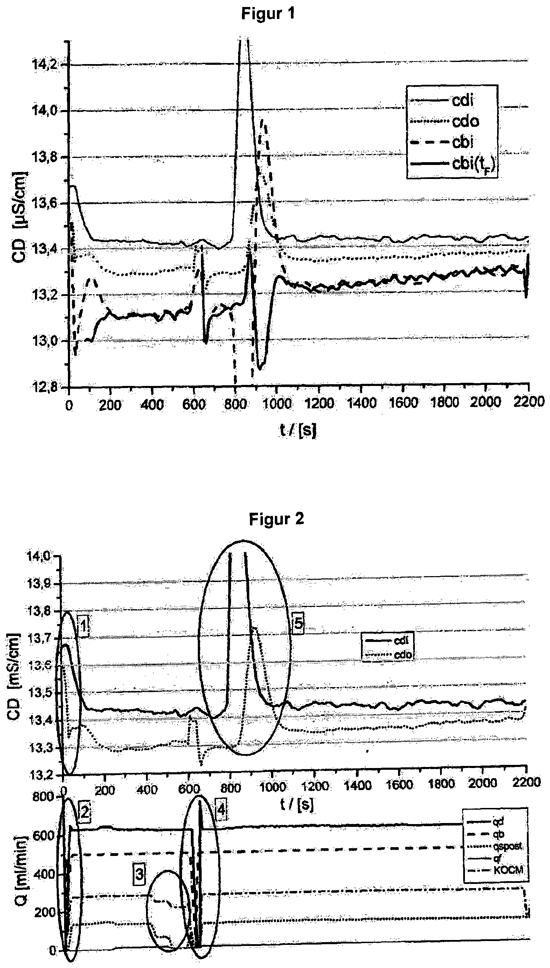

Wird eine konstante Flusszeit t_{F} zwischen den c_{di} und c_{do} ermittelnden Leitfähigkeitssensoren berücksichtigt und c_{bi} gemäß ${c}_{bi} \left({t}_{j}\right) = {c}_{di} \left({t}_{j}\right) + \frac{{Q}_{d} \left({t}_{j}\right) + {Q}_{s} \left({t}_{j}\right) + {Q}_{f} \left({t}_{j}\right)}{K \left({t}_{j}\right)} \left({c}_{do}\left({t}_{j}\right)-{c}_{di}\left({t}_{i}\right)\right)$ mit tⱼ = tᵢ + t_{F} berechnet, so werden hierdurch Variationen in c_{di}, die sich zeitlich versetzt auf c_{do} auswirken, besser berücksichtigt. Siehe in diesem Zusammenhang den Vergleich der Kurven c_{bi}(t_{F}) und c_{bi} in Figur 1.

Gerade zu Beginn der Behandlung sind c_{di} und c_{do} aber instabil. Die Leitfähigkeit c_{di} ändert sich beispielsweise wegen der Änderung der Bikarbonat-Konzentration nach Ende der Bikarbonat-Reduktion im Vorbereiten (vergleiche Figur 2, Feld Nr. 1, wo die Änderung der c_{di} in Folge der Änderung der Bikarbonat-Konzentration im Dialysat von 24 auf 32 mmol/l erkennbar ist) oder durch Verstellung durch den Anwender oder automatisierte Regelungen der Natriumionenkonzentration im Dialysat. Die Leitfähigkeit c_{do} wird durch die Kopplung mit dem Patientenblut beeinflusst. Gerade zu Beginn der Dialyse finden hierbei größere Konzentrationsänderungen durch diffusiven Austausch statt, da zu dieser Zeit die größten Gradienten vorliegen. Infusion von Spüllösung beim Konnektieren oder spätere Medikamentengaben in das Blutschlauchsystem führen ebenfalls zu kurzfristigen Änderungen von c_{do}. Wegen der Trägheit des Systems führen auch Änderungen in den Pumpenraten (z.B. Änderung der Blutpumpenrate durch den Anwender oder automatisiert zur Optimierung des Blutflusses unter Berücksichtigung des arteriellen und venösen Drucks, Anpassungen der Substituatrate durch den Anwender oder durch automatisierte Regelungen, Pumpenvariationen oder. -stopps bei der automatisierten Durchführung von Systemtests, etc.) zu zeitverzögerten Schwankungen in c_{do} (vergleiche Figur 2, Feld Nr. 2, wo die Änderung von Blut-, Dialysat-, und Substituatfluss zu Beginn der Behandlung gezeigt wird; sowie Figur 2, Feld Nr. 3, wo die automatisierte Änderung des Substituatflusses durch die Maschine gezeigt wird). Selbsttests der Maschine und Bypass-Schaltungen, bei denen der Dialysatfluss am Dialysator vorbeigeleitet wird, verhindern wegen der fehlenden Kopplung an das Patientenblut und der durch sie hervorgerufenen Instabilitäten die Bestimmung von c_{bi} (vergleiche Figur 2, Feld Nr. 4). Auch während laufender Leitfähigkeits-Variationen zur Bestimmung der Clearance (beispielsweise durch OCM, Diascan oder dgl.) ist eine Bestimmung von c_{bi} nicht möglich (vergleiche Figur 2, Feld Nr. 5). Auch durch die Änderung der Flussparameter hervorgerufene Änderungen der Clearance K(tⱼ) führen zu Schwankungen in c_{bi}.

Wird nun c_{bi} wie oben beschrieben berechnet, so führen die genannten Instabilitäten in den ersten 10 min der Dialyse zu Schwankungen im berechneten c_{bi}, die leicht Konzentrationsschwankungen von größer 5 mmol/l entsprechen können (siehe in diesem Zusammenhang den Vergleich der Kurven c_{bi}(t_{F}) und c_{bi} in Figur 1). Damit ist der so ermittelte Wert als Eingangsgröße zur Bestimmung eines Surrogats für die prädialytische Natriumionenkonzentration im Blutplasma des Dialysepatienten oder dessen Schwankung unbrauchbar.

Der vorliegenden Erfindung liegt daher die Aufgabe zugrunde, eine Möglichkeit zu finden, ein Surrogat für die prädialytische Ionenkonzentration, vorzugsweise Natriumionenkonzentration im Blutplasma ohne zusätzliche Kosten in jeder Dialysebehandlung mit hinreichender Genauigkeit angeben zu können.

Vor diesem Hintergrund betrifft die Erfindung eine Dialysemaschine mit einem extrakorporalen Blutkreislauf, einem Dialysatkreislauf, einem Dialysator und einer Recheneinheit, die sämtliche Merkmale des Anspruchs 1 aufweist, wobei im Dialysatkreislauf wenigstens ein Sensor zur Bestimmung einer Eigenschaft des Dialysats angeordnet ist. In einer nicht erfindungsgemäßen illustrativen Dialysemaschine ist vorgesehen, dass die Recheneinheit derart ausgebildet ist, dass während einer initialen Phase der Dialysebehandlung zeitliche Evaluierungsbereiche festgelegt werden, in denen alle Stabilitätskriterien aus einer vorbestimmten Gruppe erfüllt werden. Die Recheneinheit ist ferner derart ausgebildet, dass von dem wenigstens einen Sensor ermittelte Konzentration zur Bestimmung einer prädialytischen Eigenschaft des Patientenblutes herangezogen werden, wobei bei dieser Bestimmung nur solche Messwerte berücksichtigt werden, die innerhalb dieser zeitlichen Evaluierungsbereiche ermittelt wurden.

Nach dieser illustrativen Dialysemaschine werden zeitliche Bereiche innerhalb der initialen Phase der Dialysebehandlung, die außerhalb der zeitlichen Evaluationsbereiche liegen, für die Bestimmung der prädialytischen Eigenschaft des Patientenblutes also nicht herangezogen. Hierbei ist also vorgesehen, dass nicht alle in der initialen Phase der Dialysebehandlung ermittelten Messwerte herangezogen werden, sondern nur ein Teil dieser Messwerte (beispielsweise weniger als 60%, 50% oder 40% der während der initialen Phase der Dialysebehandlung ermittelten Messwerte). Zeitliche Bereiche außerhalb der initialen Phase der Dialysebehandlung werden für die Bestimmung der prädialytischen Eigenschaft des Patientenblutes vorzugsweise nicht herangezogen.

Nach derzeitigem Stand der Technik und der gängigen Praxis im Dialysebetrieb sind sowohl die Natriumionenkonzentration im Blutplasma des Patienten gegenüber anderen Elektrolyten eine bevorzugte Größe als auch die Bestimmung derselben durch Leitfähigkeitsmessungen ein bevorzugtes Verfahren. Insoweit ist in einer Ausführungsform der Erfindung vorgesehen, dass die Dialysemaschine stromaufwärts des Dialysators einen ersten Leitfähigkeitssensor und stromabwärts des Dialysators einen zweiten Leitfähigkeitssensor umfasst, und dass ermittelte Messwerte der Leitfähigkeit des Dialysats stromaufwärts und stromabwärts des Dialysators zur Bestimmung der prädialytischen Ionenkonzentration im Blutplasma des Dialysepatienten herangezogen werden, wobei bei dieser Bestimmung nur solche Leitfähigkeits-Messwerte berücksichtigt werden, die innerhalb der zeitlichen Evaluierungsbereiche ermittelt wurden. Vorzugsweise ist die zu bestimmende prädialytischen Ionenkonzentration im Blutplasma des Dialysepatienten die prädialytischen Natriumionenkonzentration im Blutplasma des Dialysepatienten. Ist auch die Bestimmung anderer prädialytischer Ionenkonzentrationen im Blutplasma des Patienten, beispielsweise der Kaliumionenkonzentration im Rahmen des erfinderischen Konzepts denkbar.

Das in der oben genannten Ausführungsform und in den Ausführungsbeispielen für Natrium und Leitfähigkeitsmessungen beschriebene Verfahren lässt sich jedoch auch auf alle anderen Substanzen erweitern, bei denen aus einer dialysatseitigen Konzentrationsmessung bei bekannter Clearance auf blutseitige Größen geschlossen werden kann. Hierzu sind dann entsprechende Sensoren nötig.

Bei der illustrativen Dialysemaschine erfolgt eine Bestimmung der prädialytischen Eigenschaft des Patientenblutes in einer initialen Phase der Behandlung, in der Änderungen der, beispielsweise, Ionenkonzentration im Blutplasma des Dialysepatienten aus physiologischen Erwägungen entweder nur innerhalb eines begrenzten Bereichs oder in weitgehend vorhersehbarer Weise erfolgt sein können. Dabei werden Bereiche im Behandlungsverlauf, in denen keine zuverlässige Bestimmung der prädialytischen Eigenschaft des Patientenblutes möglich ist, bei der Auswertung nicht berücksichtigt. Dadurch wird eine gesteigerte Genauigkeit erreicht.

Dabei kann vorgesehen sein, dass Messwerte aus mehreren und vorzugsweise allen zeitlichen Evaluierungsbereichen zur rechnerischen Abschätzung der prädialytischen Eigenschaft des Patientenblutes herangezogen werden. So kann im Rahmen einer (später näher beschriebenen) regressiven Bestimmung der prädialytischen Eigenschaft des Patientenblutes eine größere Genauigkeit erzielt werden.

Die initiale Phase der Dialysebehandlung kann nach Ablauf einer voreingestellten Behandlungsdauer enden (die Dialysebehandlung beginnt, sobald Blut durch den extrakorporalen Blutkreislauf zirkuliert und im Dialysator mit Dialysat kontaktiert wird). Ferner ist denkbar, dass die initiale Phase der Dialysebehandlung endet, wenn eine bestimmte Behandlungseffizienz erreicht ist (beispielsweise ein Kt/V-Wert von 0,3). Beispielhafte Werte umfassen eine Dauer der initialen Phase von 30 Minuten, 20 Minuten, 10 Minuten oder 5 Minuten. Die zugrundeliegende Überlegung ist, dass sich die initiale Phase der Dialysebehandlung aus der Zeit bestimmt, innerhalb derer die Änderungen der, beispielsweise, Ionenkonzentration im Blut des Dialysepatienten aus physiologischen Erwägungen entweder nur innerhalb eines begrenzten Bereichs oder in weitgehend vorhersehbarer Weise erfolgt sein können. Diese Zeit kann entsprechend aus Erfahrungswerten pauschal oder patientenspezifisch gewählt werden.

Die vorbestimmt Gruppe an Stabilitätskriterien umfasst mindestens ein Stabilitätskriterium und vorzugsweise mehrere Stabilitätskriterien.

Geeignete Stabilitätskriterien können beispielsweise durch den Abgleich einer durch Messung ermittelten Größe mit einem Schwellenwert erhalten werden. Ein Beispiel ist die Schwankung der Leitfähigkeit des Dialysats stromaufwärts und/oder stromabwärts des Dialysators. Ein weiteres Beispiel ist die Änderungsrate der Leitfähigkeit des Dialysats stromaufwärts und/oder des Dialysators (ausgedrückt z.B. als Geradensteigung im Leitfähigkeit/Zeit - Diagramm). Beispielsweise kann die Standardabweichung und/oder Änderungsrate (beispielsweise unter Heranziehung der Messwerte der vergangenen 30 oder 60 Sekunden) mit einem Schwellenwert verglichen werden. Ist die Standardabweichung und/oder Änderungsrate geringer als der Schwellenwert, ist dieses Stabilitätskriterium erfüllt und der Zeitraum, in dem die jüngsten Messwerte erhalten wurden (z.B. die vergangenen 30 oder 60 Sekunden), fällt vorbehaltlich der Erfüllung etwaiger weiterer Stabilitätskriterien in einen zeitlichen Evaluationsbereich. Die in diesem Zeitraum erhaltenen Messwerte werden zur Bestimmung der prädialytischen Ionenkonzentration im Blutplasma des Dialysepatienten herangezogen. Ist die Standardabweichung und/oder Änderungsrate höher als der Schwellenwert, ist dieses Stabilitätskriterium nicht erfüllt und der Zeitraum, in dem die jüngsten Messwerte erhalten wurden, fällt aus dem zeitlichen Evaluationsbereich. Die Messwerte werden zur Bestimmung nicht herangezogen.

Ferner eignet sich der zeitliche Abstand zu bestimmten Ereignissen als Stabilitätskriterium. Ein Stabilitätskriterium kann beispielsweise erfüllt sein, wenn eine gewisse Sperrzeit nach einem gewissen Ereignis abgelaufen ist. Die Sperrzeit fällt aus dem zeitlichen Evaluationsbereich. Die in diesem Zeitraum erhaltenen Messwerte werden zur Bestimmung der prädialytischen Ionenkonzentration im Blutplasma des Dialysepatienten nicht herangezogen. Die nach Ablauf der Sperrzeit erhaltenen Messwerte werden vorbehaltlich der Erfüllung etwaiger weiterer Stabilitätskriterien zur Abschätzung herangezogen.

Beispiele für Ereignisse, die eine Sperrzeit auslösen können, umfassen Änderungen des Dialysatflusses, des Blutflusses, des Substituatfluss, der UF Rate, der Dialysatzusammensetzung (Natriumionen- oder Bicarbonatkonzentration, Wechsel des Konzentrats, etc.). Die Änderungen können durch geänderte Vorgaben durch den Anwender oder durch automatisierte Einstellungen hervorgerufen werden. Weitere Beispiele für Ereignisse, die eine Sperrzeit auslösen können, umfassen Bypass-Schaltungen und Pumpenstops, beispielsweise bei Infusion von Spüllösung in den extrakorporalen Blutkreislauf, Selbsttests des Systems (Druckhaltetest) oder in Folge von Anwenderaktionen (Öffnen von Türen und Abdeckungen). Die Sperrzeit kann kleiner als 2 Minuten betragen, beispielsweise zwischen 15 und 90 Sekunden oder zwischen 30 und 60 Sekunden. Sie kann für unterschiedliche Ereignisse unterschiedlich gewählt werden. Beispielsweise kann die Sperrzeit nach einer Bypass-Schaltung 60 Sekunden und nach einer Änderung des Dialysatflusses 30 Sekunden betragen. Auch das Änderungsausmaß kann berücksichtigt werden (z.B. 30 Sekunden bei Änderung des Dialysatflusses um kleiner 300 ml/min und 60 Sekunden bei Änderung um größer 300 ml/min).

Vor dem eingangs genannten Hintergrund betrifft die Erfindung eine Dialysemaschine mit einem extrakorporalen Blutkreislauf, einem Dialysatkreislauf, einem Dialysator und einer Recheneinheit, wobei im Dialysatkreislauf stromaufwärts des Dialysators ein erster Sensor und stromabwärts des Dialysators ein zweiter Sensor angeordnet ist. Erfindungsgemäß ist die Recheneinheit derart ausgebildet, dass ermittelte Messwerte stromaufwärts des Dialysators zu einem ersten Zeitpunkt und stromabwärts des Dialysators zu einem späteren zweiten Zeitpunkt als korrespondierende Wertepaare zur Bestimmung einer prädialytischen Eigenschaft des Patientenblutes herangezogen werden, wobei der zeitliche Versatz zwischen dem ersten und zweiten Zeitpunkt an die Flusszeit des Dialysats zwischen erstem und zweitem Sensor angenähert wird oder dieser entspricht.

Insoweit wird erfindungsgemäß bei der Bestimmung der prädialytischen Eigenschaft des Patientenblutes die Flusszeit des Dialysats zwischen den beiden Leitfähigkeitssensoren berücksichtigt. Treten in der Leitfähigkeit der Dialyselösung größere Schwankungen auf, so kann durch den Zeitversatz, den die Dialyselösung zum Durchlaufen des Dialysators braucht, ein Fehler bei der Bestimmung der prädialytischen Eigenschaft des Patientenblutes entstehen, weil die Differenz nicht mehr korrekt die Konzentrationsänderung im Filter wiederspiegelt. Dieser Fehler wird durch die Berücksichtigung der Flusszeit vermindert.

In einer Ausführungsform handelt es sich bei dem ersten und zweiten Sensor um Leitfähigkeitssensoren und die Recheneinheit ist derart ausgebildet, dass es sich bei den ermittelten Messwerten um die Leitfähigkeiten des Dialysats stromaufwärts und stromabwärts des Dialysators handelt, und dass diese Leitfähigkeiten als korrespondierende Wertepaare zur Bestimmung der prädialytischen Ionenkonzentration, vorzugsweise Natriumionenkonzentration im Blutplasma des Dialysepatienten herangezogen werden.

In einer Ausführungsform ist vorgesehen, dass die erfindungsgemäße Maßnahme der Berücksichtigung der Flusszeit des Dialysats zwischen den beiden Sensoren und die erfindungsgemäße Maßnahme der Auswahl zeitlicher Evaluierungsbereiche innerhalb der initialen Behandlungsphase anhand von Stabilitätskriterien kombiniert zum Einsatz kommen.

Der zeitliche Versatz wird vorzugsweise angepasst, sofern sich die Flussgeschwindigkeit des Dialysats im Verlauf der Behandlung ändert.

Der zeitliche Versatz kann in einer Ausführungsform aus dem Volumen des hydraulischen Systems zwischen den beiden Sensoren (Volumen der Leitungsabschnitte und der Dialysatkammer des Dialysators) und dem Dialysatfluss in Volumen pro Zeit berechnet werden.

Alternativ kann der zeitliche Versatz anhand der zeitlichen Differenz zwischen der Detektion einer Störung (beispielsweise kurze Konzentrationserhöhung) am ersten und zweiten Sensor ermittelt werden. In diesem Fall kann vorgesehen sein, dass nach einer Änderung der Flussgeschwindigkeit des Dialysats keine neue Bestimmung des zeitlichen Versatzes durch Detektion einer Störung erfolgt, sondern dass der zeitliche Versatz unter Berücksichtigung der alten und neuen Flussgeschwindigkeit durch Extrapolation aktualisiert wird.

In einer Ausführungsform zur Bestimmung der prädialytischen Ionenkonzentration im Blutplasma des Dialysepatienten ist vorgesehen, dass anhand der stromaufwärts und stromabwärts des Dialysators ermittelten Leitfähigkeitswerte eine prädialytische Plasma-Äquivalent-Leitfähigkeit bestimmt wird, und dass aus der prädialytischen Plasma-Äquivalent-Leitfähigkeit in weiterer Folge die Ionenkonzentration im Plasma des Dialysepatienten bestimmt wird. Dies kann anhand der eingangs und in weiterer Folge im Ausführungsbeispiel genannten mathematischen Operationen erfolgen.

In einer Ausführungsform zur Bestimmung der prädialytischen Ionenkonzentration im Blutplasma des Dialysepatienten ist vorgesehen, dass die prädialytische Plasma-Äquivalent-Leitfähigkeit durch Extrapolation momentaner Plasma-Äquivalent-Leitfähigkeiten bestimmt wird, die für die zeitlichen Evaluierungsbereiche und/oder unter Berücksichtigung des zeitlichen Versatzes aus den Leitfähigkeitswerten stromaufwärts und stromabwärts des Dialysators bestimmt werden. Dabei kann beispielsweise eine zeitabhängige Interpolation vorgesehen sein, wobei die Regression der Daten als Funktion der Zeit auf ein Polynom der Ordnung n erfolgen kann. Bevorzugte Ordnungen sind n=0 (Mittelwertbildung) und n=1 (lineare Regression). Eine andere Möglichkeit liegt in der Verwendung einer nichtlinearen Funktion, z.B. in der Modellierung eines exponentiellen Anstiegs oder Abfalls der Natriumionenkonzentration als Funktion der Zeit. Statt einer zeitabhängigen Interpolation kann auch eine Interpolation basierend auf einem anderen Parameter vorgesehen sein, beispielsweise eine Regression der Daten als Funktion des Kt/V-Wertes. Dies kann anhand der eingangs und in weiterer Folge im Ausführungsbeispiel genannten mathematischen Operationen erfolgen.

Alternativ ist zur Bestimmung der prädialytischen Ionenkonzentration im Blutplasma des Dialysepatienten denkbar, dass die prädialytische Ionenkonzentration durch Extrapolation momentaner Ionenkonzentrationen bestimmt wird, die für die zeitlichen Evaluierungsbereiche und/oder unter Berücksichtigung des zeitlichen Versatzes aus den Leitfähigkeitswerten stromaufwärts und stromabwärts des Dialysators bestimmt werden.

Die Offenbarung überlegt ferner ein Dialyseverfahren, das anhand einer erfindungsgemäßen Dialysemaschine durchgeführt werden kann und die Schritte der in der Recheneinheit hinterlegten Routine abarbeitet.

Bei der erfindungsgemäßen Dialysemaschine kann es sich beispielsweise um eine solche für die Hämodialyse, die Hamodiafiltration oder die Hämofiltration handeln.

Weitere Einzelheiten und Vorteile der Erfindung ergeben sich aus den beigefügten Figuren und anhand der nachfolgend beschriebenen Ausführungsbeispiele. In den Figuren zeigen:
Figur 1:
   ein Diagramm der berechneten Plasma-Leitfähigkeit mit und ohne Berücksichtigung der Flusszeit t_{F};
Figur 2:
   ein Diagramm des zeitlichen Verlaufs von Leitfähigkeiten, Flüssen und berechneter Clearance;
Figur 3:
   eine schematische Darstellung einer erfindungsgemäßen Dialysemaschine mit der Fähigkeit zur Abschätzung der prädialytischen Natriumionenkonzentration im Blutplasma eines Dialysepatienten;
Figur 4:
   ein Diagramm, welches einen Fit der Plasma-Leitfähigkeit bei Auftragung gegen die Behandlungsdauer zeigt; und
Figur 5:
   ein Diagramm, welches einen Fit der Plasma-Leitfähigkeit bei Auftragung gegen die Dialysedosis Kt/V zeigt.

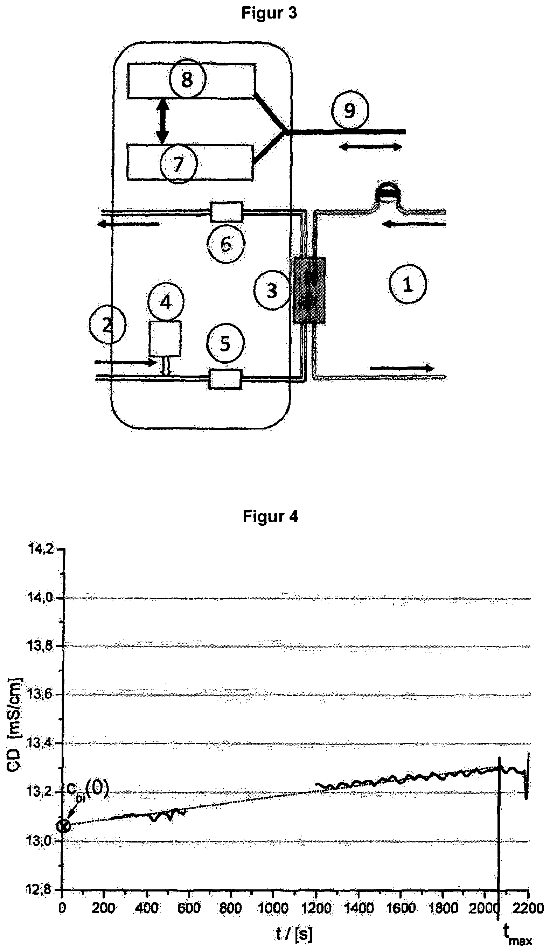

Figur 3 zeigt eine schematische Darstellung einer Ausführungsform einer erfindungsgemäßen Dialysemaschine mit der Fähigkeit zur Abschätzung der prädialytischen Natriumionenkonzentration im Blutplasma eines Dialysepatienten.

Dabei steht ein Blutkreislauf 1 über einen Dialysator 3 mit einem Dialysatkreislauf 2 in Verbindung. Der Dialysatkreislauf 2 umfasst eine Konzentrat-Dosiereinheit 4 sowie in der Figur nicht näher dargestellte Pumpen, Ventile und Sensoren. Die Messung der temperaturkompensierten dialysatseitigen Leitfähigkeit stromaufwärts und stromabwärts des Dialysators erfolgt mit ersten und zweiten Leitfähigkeitszellen 5 und 6. Flusssensoren und die Leitfähigkeitszellen übermitteln kontinuierlich Messdaten an die Recheneinheit 7. In dieser sind die in weiterer Folge beschriebenen Algorithmen zur Bestimmung der prädialytischen Natriumionenkonzentration im Blutplasma des Dialysepatienten hinterlegt. Die Recheneinheit 7 steht in Verbindung mit einem Benutzerinterface 8 zur Benachrichtigung des Anwenders. Daten aus der Recheneinheit 7 bzw. dem Benutzerinterface 8 können über ein Datennetzwerk 9 zur weiteren Speicherung und Verarbeitung an einen externen Rechner übermittelt werden.

Der in der Recheneinheit 7 hinterlegte Algorithmus umfasst die folgenden Elemente:

### Berechnung von c_{bi} unter Berücksichtigung der Verzögerungszeit t_{F}

Die Verzögerungszeit t_{F}(Q_{d}) kann bei Kenntnis der hydraulischen Eigenschaften des Systems zwischen den Leitfähigkeitszellen 5 und 6 und des Dialysatflusses bei konstantem Dialysatfluss Q_{d} unmittelbar berechnet werden. Die im Dialysator 6 durch dessen Volumen erfolgende Verzögerung kann aus dem Dialysatortyp entnommen werden, der entweder manuell oder automatisch erkannt wird (z.B. durch Markierung des Dialysators mittels RFID-Tag oder Barcode und Auslesen mit entsprechender Einheit). Alternativ kann t_{F} aus der zeitlichen Verzögerung der Antwort in c_{do} auf eine Leitfähigkeitsänderung in c_{di} (z.B. Leitfähigkeitspuls zur Bestimmung der Clearance, vergleiche Figur 1) erfolgen. Weicht zu einem Zeitpunkt tⱼ Q_{d}(tⱼ) vom bei der Bestimmung von t_{F} zum Zeitpunkt t_{M} vorliegenden Dialysatfluss ab, so kann t_{F}(Q_{d}(tⱼ)) durch Extrapolation bestimmt werden z.B. durch ${t}_{F} \left({t}_{J}\right) = {t}_{F} \frac{{Q}_{d} \left({t}_{M}\right)}{{Q}_{d} \left({t}_{J}\right)}$ [5].

Anschließend wird c_{bi} gemäß der eingangs dargestellten Formel 4 berechnet. Hierbei ist zu berücksichtigen, dass aus Kapazitätsgründen die Speicherung der relevanten Daten nur in zeitlichen Abständen Δtₛ erfolgt. Eine Verbesserung der Berechnung von c_{bi} ist daher durch Verkürzung von Δtₛ in der für die Berechnung des initialen Plasma-Na benötigten Zeitintervalls auf ein vertretbares Minimum zu erreichen. Alternativ kann eine Interpolation von Zwischenwerten für c_{bi} auf Basis der benachbarten gespeicherten Daten erfolgen.

### Elimination von Bereichen, in denen keine zuverlässige Berechnung von c_{bi} möglich ist

Zeitliche Bereiche, in denen das wie oben beschrieben Berechnete aufgrund verschiedener Stabilitätskriterien nicht dem realen Wert entspricht, werden für die weitere Auswertung nicht berücksichtigt. Hierzu gehören folgende Stabilitätskriterien:
- Änderungen in den Vorgaben durch den Anwender oder durch automatisierte Einstellungen an Dialysat, Blut- und Substituatfluss sowie der Ultrafiltrationsrate oder an der Dialysatzusammensetzung (Sollwerte für Natrium und Bikarbonat, Wechsel des Konzentrats, etc.);
- Bypass-Schaltungen und Pumpenstops bei Selbsttests des Systems oder in Folge von Anwenderaktionen (z.B. Öffnen von Türen und Abdeckungen).

Bei Änderungen wird ab dem Zeitpunkt der Änderung c_{bi} für eine Dauer t_{D_change,j} als ungültig markiert. t_{D_change,j} ist in der Recheneinheit 7 hinterlegt und kann je nach Art der Störung unterschiedliche Werte annehmen (z.B. 1 Minute nach Bypass-Schaltung, 30 Sekunden nach Änderung der Blutpumpenrate). Auch können Regeln hinterlegt sein, nach denen t_{D_Change,j} vom Ausmaß der Änderung eines Parameters abhängt (z.B. 30 Sekunden bei Änderung des Dialysatflusses um 100 ml/min, 60 Sekunden bei Änderung um >300 ml/min).

Des Weiteren kann eine nicht hinreichende Stabilität von c_{di} und c_{do} als Auslöser für eine Sperrzeit t_{D_stab,j} verwendet werden. Damit kann c_{bi} für so lange als ungültig markiert werden, bis wieder eine hinreichende Stabilität vorliegt. Folgende Stabilitätskriterien für Instabilität können hierbei angewendet werden:
- Schwankung der LF (c_{di} bzw. c_{do}),. ausgedrückt z.B. als Standardabweichung, oberhalb eines vordefinierten Schwellwerts;
- Änderungsrate der LF, ausgedrückt z.B. als Geradensteigung, oberhalb eines vordefinierten Schwellwerts.

Nach Elimination der als ungültig markierten Werte von c_{bi} verbleiben im Speicher der Recheneinheit die zur weiteren Auswertung verwendbaren Werte (vergleiche Figur 4, durchgezogene Linie).

### Extrapolation von c_{bi} auf den prädialvtischen Wert

Zur Extrapolation von c_{bi} auf den prädialytischen Wert werden alle verbleibenden Werte von c_{bi} bis hin zu einer maximalen initialen Dialysedauer tₘₐₓ verwendet. Die maximale initiale Dialysedauer tₘₐₓ bestimmt sich aus der Zeit, innerhalb derer die Änderungen der Konzentration der Natriumionen im Blutplasma des Dialysepatienten aus physiologischen Erwägungen entweder nur innerhalb eines begrenzten Bereichs oder in weitgehend vorhersehbarer Weise erfolgt sein können, z.B. bei Modellierung des Stoffaustauschs zwischen Blut und Dialysat durch ein 1-Pool-Modell wie in der nachfolgend dargestellten Formel 6: ${c}_{bi} \left(t\right) = {c}_{di} + \left({c}_{bi}\left(0\right)-{c}_{di}\right) {e}^{- \frac{Kt}{V}}$ [6]

Die maximale initiale Dialysedauer tₘₐₓ kann dabei z.B. eine feste Zeit, z.B. 30 Minuten sein, oder der Zeitpunkt, bis zu dem eine bestimmte Behandlungseffizienz, z.B. Kt/V=0,3 erreicht ist.

Die Interpolation erfolgt dann z.B. durch Regression mit einem Polynom der Ordnung n. Bevorzugte Ordnungen sind n=0 (Mittelwertbildung) und n=1 (lineare Regression) mit den verbleibenden Stützstellen (vergleiche Figur 4, gepunktete Linie). Eine andere Möglichkeit ist die Extrapolation mittels einer nichtlinearen Funktion, z.B. die Modellierung eines exponentiellen Anstiegs oder Abfalls von c_{bi}.

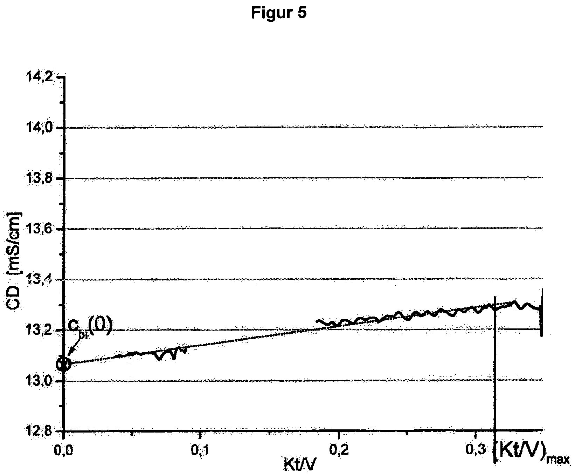

Statt einer zeitlichen Interpolation kann auch eine Interpolation basierend auf einem Modell für die Änderung von c_{bi} wie in Formel 6 erfolgen (vergleiche Figur 5). Auch hier ist eine polynombasierte Regression möglich: In erster Näherung gilt: e^{-x} ≈ 1-x, wobei die Abweichung für x<0,3 weniger als 5% beträgt. Damit gilt für Kt/V<0,3 in guter Näherung: ${c}_{bi} \left(t\right) = {c}_{bi} \left(0\right) + \left({c}_{di}-{c}_{bi}\left(0\right)\right) \left(\frac{Kt}{V}\right)$ [7]

Damit kann c_{bi}(0) auch durch linearen Fit einer Auftragung gegen Kt/V bestimmt werden (vergleiche Figur 5). Hierdurch können Änderungen der die Clearance bestimmenden Flüsse besser berücksichtigt werden.

### Umrechnung auf die prädialytische Natriumionenkonzentration im Blutplasma des Dialysepatienten

Die wie eben beschrieben bestimmte prädialytische Plasma-Äquivalent-Leitfähigkeit c_{bi}(0) kann nun mittels eines Modells für den Zusammenhang zwischen temperaturkompensierter Leitfähigkeit und Elektrolytzusammensetzung auf eine prädialytische Natriumionenkonzentration im Blutplasma des Dialysepatienten ĉ^{Na}_{bi}(0) umgerechnet werden. $\begin{matrix}{{c}^{˜}}_{bi}^{Na} \left(0\right) = f \left({c}_{bi}\left(0\right),{{c}^{˜}}_{bi}^{J}\left(0\right)\right) \\ = {a}_{0} + {a}_{i} {c}_{bi} \left(0\right) + {\sum }_{j} {b}_{j} {{c}^{˜}}_{bi}^{J} \left(0\right)\end{matrix}$ [8] ĉ^{j}_{bi}(0) bezeichnet die Konzentration anderer Elektrolyte als Na, z.B. Kalium, die einen Einfluss auf die Leitfähigkeit haben. Deren Konzentration kann durch vom Anwender durch eine Blutanalyse bestimmt und manuell über das Benutzerinterface 8 oder eine Datenverbindung 9 mit einem externen Speichermedium oder einer Datenbank eingegeben werden. Durch Kenntnis von ĉ^{j}_{bi}(0) ist eine höhere Genauigkeit erreichbar, wobei im Allgemeinen die Annahme von Standardwerten hinreichend ist. Die Faktoren a₀, a₁ und bⱼ sind hierbei fest in der Recheneinheit hinterlegt.

### Speicherung und Trendanalvse

Die prädialytische Natriumionenkonzentration im Blutplasma des Dialysepatienten ĉ^{Na}_{bi}(0) kann nun am Benutzerinterface 8 angezeigt oder über die Datenverbindung 9 an ein externes Speichermedium oder eine Datenbank übergeben werden. Aus einer Trendanalyse der aktuellen und vergangenen Bestimmungen von ĉ^{Na}_{bi}(0) kann der Anwender über systematische Trends der prädialytischen Natriumionenkonzentration im Blutplasma des Dialysepatienten und deren Schwankung informiert werden. Durch Vergleich der ermittelten Änderungsrate und der Schwankung von ĉ^{Na}_{bi}(0) mit hinterlegten Referenzwerten kann der Anwender beim Überschreiten vordefinierter Grenzen über kritische Werte dieser Größen informiert werden.

Durch den in der Recheneinheit hinterlegten Algorithmus und durch die weiteren oben beschriebenen baulichen Merkmale umfasst die oben näher beschriebene erfindungsgemäße Dialysemaschine zur Bestimmung der prädialytischen Natriumionenkonzentration eines Dialysepatienten also unter anderem folgende Fähigkeiten:
Mittels der Recheneinheit kann aus darin hinterlegten Datensätzen, bestehend aus Leitfähigkeiten und Flüssen und Informationen zu Störungen des Dialyseregimes (z.B. Bypass-Schaltungen), in der initialen Phase der Dialyse (z.B., < 10 min) ein Bereich gesucht werden, in dem stabile Leitfähigkeiten und Dialysebedingungen vorliegen. Hierzu werden in der Recheneinheit verschiedene Stabilitätskriterien (z.B. zeitlicher Abstand zu letzter Konzentrationsänderung oder Änderung der Pumpenförderrate, Standardabweichung oder Steigung der Leitfähigkeit) hinterlegt und ausgewertet. Innerhalb dieses Bereichs kann eine Mittelung der c_{di} und c_{do} zur Verringerung von Fluktuationen erfolgen. Der auf Grund der hydraulischen Flussstrecken vorliegende zeitliche Versatz zwischen c_{di} und c_{do} kann berücksichtigt werden, indem bei der Berechnung von c_{bi} die Werte von c_{di} zum Zeitpunkt t, von c_{do} aber zum Zeitpunkt t+t_{F} verwendet werden, wobei t_{F} der Flusszeit des Dialysats zwischen den beiden Leitfähigkeitssensoren entspricht. Ist wegen instabiler Dialyseverhältnisse eine Bestimmung von c_{bi} innerhalb der ersten 5 Minuten der Dialysebehandlung nicht möglich, so können für c_{bi} innerhalb der ersten 30 Minuten der Dialysebehandlung Stützstellen zu Zeiten stabiler Dialysebedingungen bestimmt werden und eine Extrapolation auf den initialen Wert zu Dialysebeginn durchgeführt werden. Der so ermittelte Wert des initialen c_{bi} wird mittels eines Elektrolyt-Modells in ein eine prädialytische Natriumionenkonzentration im Blutplasma des Dialysepatienten umgerechnet, da ĉ^{Na}_{bi}(0) = f (c_{bi}). Die prädialytische Natriumionenkonzentration im Blutplasma des Dialysepatienten ĉ^{Na}_{bi}(0) kann spätestens am Ende der Dialysebehandlung auf einem internen oder einem externen Speichermedium (Patientenkarte, Übertragung über Netzwerk, etc.) patientenbezogen gespeichert werden. Zusammen mit den ĉ^{Na}_{bi}(0) aus früheren Behandlungen kann die Variabilität von ĉ^{Na}_{bi}(0) berechnet und als Anzeigeparameter dem Arzt zur Verfügung gestellt werden. Statt am Ende der Dialyse kann ĉ^{Na}_{bi}(0) und dessen Variabilität unmittelbar nach dessen Berechnung angezeigt werden. Dieses ist im Allgemeinen sofort nach der ersten erfolgreichen OCM-Messung (nach etwa 20-miütiger Behandlungsdauer) möglich. Bei Verzögerung der ersten erfolgreichen OCM-Messung kann dies aber auch erst später möglich sein.

Nach derzeitigem Stand der Technik und der gängigen Praxis im Dialysebetrieb sind sowohl die prädialytische Natriumionenkonzentration im Blutplasma des Dialysepatienten gegenüber anderen Elektrolyten eine bevorzugte Größe als auch die Bestimmung derselben durch Leitfähigkeitsmessungen ein bevorzugtes Verfahren. Eine darauf aufbauende Ausführungsform der erfindungsgemäßen Dialysemaschine wurde oben beschrieben. Jedoch lässt sich das für Natrium und LF- Messung beschriebene Verfahren auch auf alle anderen Substanzen erweitern, bei denen aus einer dialysatseitigen Konzentrationsmessung bei bekannter Clearance auf blutseitige Größen geschlossen werden kann. Hierzu sind dann entsprechende Sensoren nötig. Ist die entsprechende Substanz schon im zufließenden Dialysat vorhanden, so müssten die Leitfähigkeitssensoren 5 und 6 durch Sensoren ersetzt werden, die spezifisch die Konzentration der Substanz bestimmen, deren prädialytische Plasmakonzentration bestimmt werden soll.

Hierzu können z.B. ionenselektive Elektroden zur Messung der Konzentration von Kalium, Calcium, Magnesium und Chlorid verwendet werden. Aber auch andere Messverfahren zur Bestimmung von Elektrolyten, z.B. auch durch NMR, sind denkbar. Sensor 5 kann entfallen, wenn die Konzentration stromauf des Dialysators dadurch bestimmt wird, dass zu einem bestimmten Zeitpunkt eine Bypass-Schaltung stattfindet, mit der das frische Dialysat direkt mit Sensor 6 gemessen werden kann, unter der Voraussetzung, dass sichergestellt ist, dass sich die betreffende Konzentration während der zur Bestimmung des prädialytischen Plasmawerts benötigten Zeit nicht wesentlich ändert. Ebenfalls kann der Wert im frischen Dialysat bereits durch Herstellerangaben und genaue Kenntnis des Mischsystems bekannt sein, so dass eine kontinuierliche Bestimmung stromaufwärts des Dialysators entfallen kann. Insbesondere bei Substanzen, die nicht im frischen Dialysat vorhanden sind, kann Sensor 5 entfallen. Bei Kenntnis der Clearance dieser Substanzen, z.B. aus der leitfähigkeitsbasierten Bestimmung der Dialysatorclearance und Approximation auf die Clearance des entsprechenden Stoffes mittels eines hinterlegten Korrekturfaktors, kann nach dem oben beschriebenen Verfahren deren prädialytische Konzentration bestimmt werden. Sensor 6 kann dabei auch eine spektroskopische Größe wie z.B. die Absorption oder die Fluoreszenz bestimmen, wobei in der Auswerteeinheit ein Berechnungsverfahren hinterlegt ist, dass aus den spektralen Messungen auf Stoffkonzentrationen schließt. Zur Bestimmung der Glucosekonzentration können Sensoren verwendet werden, die die Drehung der Polarisationsrichtung von polarisiertem Licht beim Durchtritt durch eine die Probelösung enthaltende Messstrecke bestimmen. Alternativ kann die Änderung des Brechungsindex durch Refraktometrie bestimmt werden. Wie oben muss dann in der Auswerteeinheit ein Berechnungsverfahren hinterlegt sein, mit dessen Hilfe auf Stoffkonzentrationen geschlossen werden kann. Schwankungen in der Blutglucose können ein wichtiger Indikator für eine unzureichende Diabetestherapie sein. Weiterhin können statt der Sensoren 5 und 6 an deren Positionen mehrere Sensoren eingesetzt werden, mit deren Hilfe gemäß dem beschriebenen Verfahren die prädialytische Konzentration unterschiedlicher Substanzen bestimmt werden kann. Eine für einen ersten Parameter bestimmte prädialytische Konzentration kann dann zur Verbesserung der Genauigkeit in der Bestimmung der Konzentration eines weiteren Parameters wie oben beschrieben verwendet werden.

Zusammenfassend ergibt sich, dass die Natriumkonzentration im Blutplasma ein wichtiger diagnostischer Parameter bei Dialysepatienten ist. Die Bestimmung dieses Wertes durch Blutanalysen ist aber aufwendig und teuer, weshalb nach Alternativen gesucht wurde. Die Berechnung der Konzentration verschiedener Stoffe in Blut über Leitfähigkeitsmessungen auf Dialysatseite im extrakorporalen Kreislauf wird im Stand der Technik bereits beschrieben. Diese Berechnung der prädialytischen Blutkonzentration beruht dann auf der Extrapolation dieser Werte. Verlässliche Messungen liegen aber erst nach circa 20 Minuten ab Behandlungsbeginn vor. Messungen zu Beginn der Behandlung sind großen Schwankungen unterworfen, die zu größeren Fehlern bei der Konzentrationsermittlung führen. Diese Fehler werden durch die Erfindung vermindert. Erfindungsgemäß werden zum einen Bereiche im Behandlungsverlauf, in denen keine zuverlässige Berechnung möglich ist, bei der Auswertung nicht berücksichtigt. Zum anderen wird bei der Berechnung der Plasmakonzentration die Flusszeit des Dialysates zwischen den beiden Leitfähigkeitssensoren berücksichtigt. Im Stand der Technik werden in die Berechnungsformel die Ergebnisse der Leitfähigkeitsmessungen, die gleichzeitig stattfinden, eingesetzt. Treten in der Leitfähigkeit der Dialyselösung aber größere Schwankungen auf, so entsteht durch den Zeitversatz, den die Dialyselösung zum Durchlaufen des Filters braucht, ein Fehler, weil die Differenz nicht mehr korrekt die Konzentrationsänderung im Filter wiederspiegelt. Dieser Fehler wird durch die Berücksichtigung der Flusszeit vermindert.

## Patentansprüche

1. Dialysemaschine mit einem extrakorporalen Blutkreislauf, einem Dialysatkreislauf, einem Dialysator und einer Recheneinheit, wobei im Dialysatkreislauf stromaufwärts des Dialysators ein erster Sensor und stromabwärts des Dialysators ein zweiter Sensor angeordnet ist,
**dadurch gekennzeichnet,**
**dass** die Recheneinheit derart ausgebildet ist, dass ermittelte Messwerte stromaufwärts des Dialysators zu einem ersten Zeitpunkt und stromabwärts des Dialysators zu einem späteren zweiten Zeitpunkt als korrespondierende Wertepaare zur Bestimmung einer prädialytischen Eigenschaft des Patientenblutes herangezogen werden, wobei der zeitliche Versatz zwischen dem ersten und zweiten Zeitpunkt an die Flusszeit des Dialysats zwischen erstem und zweitem Sensor angenähert wird oder dieser entspricht.

2. Dialysemaschine nach Anspruch 1, **dadurch gekennzeichnet, dass** es sich bei dem ersten und zweiten Sensor um Leitfähigkeitssensoren handelt, und dass die Recheneinheit derart ausgebildet ist, dass es sich bei den ermittelten Messwerten um die Leitfähigkeiten des Dialysats stromaufwärts und stromabwärts des Dialysators handelt, und dass diese Leitfähigkeiten als korrespondierende Wertepaare zur Bestimmung der prädialytischen Ionenkonzentration, vorzugsweise Natriumionenkonzentration im Blutplasma des Dialysepatienten herangezogen werden.

3. Dialysemaschine nach Anspruch 1 oder 2, **dadurch gekennzeichnet, dass** die Recheneinheit ferner derart ausgebildet ist, dass der zeitliche Versatz aus dem Volumen des hydraulischen Systems zwischen den beiden Sensoren und dem Dialysatfluss berechnet wird, oder dass der zeitliche Versatz anhand der zeitlichen Differenz zwischen der Detektion einer Störung am ersten und zweiten Sensor ermittelt wird.

4. Dialysemaschine nach einem der Ansprüche 1 bis 3, **dadurch gekennzeichnet, dass** die Recheneinheit ferner derart ausgebildet ist, dass der zeitliche Versatz angepasst wird, sofern sich die Flussgeschwindigkeit des Dialysats im Verlauf der Behandlung ändert.

5. Dialysemaschine nach einem der Ansprüche 1 bis, **dadurch gekennzeichnet, dass** die Recheneinheit ferner derart ausgebildet ist, dass anhand der stromaufwärts und stromabwärts des Dialysators ermittelten Leitfähigkeitswerte eine prädialytische Plasma-Äquivalent-Leitfähigkeit bestimmt wird, und dass aus der prädialytischen Plasma-Äquivalent-Leitfähigkeit in weiterer Folge die Ionenkonzentration im Plasma des Dialysepatienten bestimmt wird.

6. Dialysemaschine nach Anspruch 5, **dadurch gekennzeichnet, dass** die Recheneinheit ferner derart ausgebildet ist, dass die prädialytische Plasma-Äquivalent-Leitfähigkeit durch Extrapolation momentaner Plasma-Äquivalent-Leitfähigkeiten bestimmt wird, die für die zeitlichen Evaluierungsbereiche und/oder unter Berücksichtigung des zeitlichen Versatzes aus den Leitfähigkeitswerten stromaufwärts und stromabwärts des Dialysators bestimmt werden, wobei die Extrapolation vorzugsweise eine Regression der momentanen Plasma-Äquivalent-Leitfähigkeiten als Funktion der Zeit oder als Funktion des Kt/V-Wertes umfasst.

## Claims

1. A dialysis machine, having an extracorporeal blood circuit (1), a dialyzate circuit (2), a dialyzer (3) and a processing unit (7), wherein a first sensor is arranged upstream of the dialyzer in the dialyzate circuit and a second sensor is arranged downstream of the dialyzer,
**characterised in that**
the processing unit is configured such that determined measured values upstream of the dialyzer at a first time and determined measured values downstream of the dialyzer at a later second time are used as corresponding value pairs for determining a pre-dialysis property of the patient's blood, wherein the time offset between the first and second times being approximated to the flow time of the dialyzate between the first and second sensors, or corresponds thereto.

2. The dialysis machine in accordance with claim 1, **characterized in that** the first and second sensors are conductivity sensors; and **in that** the processing unit is configured such that the determined measured values are the conductivities of the dialyzate upstream and downstream of the dialyzer; and **in that** these conductivities are used as corresponding value pairs for determining the predialytic ion concentration, preferably the sodium ion concentration, in the blood plasma of the dialysis patient.

3. The dialysis machine in accordance with claim 1 or 2, **characterized in that** the processing unit is furthermore configured such that the time offset is calculated from the volume of the hydraulic system between the two sensors and the dialysis flow; or **in that** the time offset is determined by means of the time difference between the detection of a disturbance at the first and second sensors.

4. The dialysis machine in accordance with one of the claims 1 to 3, **characterized in that** the processing unit is furthermore configured such that the time offset is adapted, provided that the flow speed of the dialyzate changes in the course of the treatment.

5. The dialysis machine in accordance with one of the claims 1 to 4, **characterized in that** the processing unit is furthermore configured such that a predialytic plasma-equivalent conductivity is determined by means of the conductivity values determined upstream and downstream of the dialyzer; and **in that** subsequently the ion concentration in the plasma of the dialysis patient is determined from the predialytic plasma-equivalent conductivity.

6. The dialysis machine in accordance with claim 5, **characterized in that** the processing unit is furthermore configured such that the predialytic plasma-equivalent conductivity is determined by extrapolation of instantaneous plasma-equivalent conductivities which are determined for the temporal evaluation ranges and/or while considering the time offset from the conductivity values upstream and downstream of the dialyzer, wherein the extrapolation preferably comprises a regression of the instantaneous plasma-equivalent conductivities as a function of the time or as a function of the Kt/V value.

## Revendications

1. Machine de dialyse avec un circuit de sang extracorporel (1), un circuit de dialysat (2), un dialyseur (3) et une unité de calcul (7), dans laquelle dans le circuit de dialysat, un premier capteur est disposé en amont du dialyseur et un deuxième capteur est disposé en aval du dialyseur,
**caractérisée en ce que**
l'unité de calcul est réalisée de telle manière que des valeurs de mesure déterminées en amont du dialyseur à un premier moment et en aval du dialyseur à un deuxième moment ultérieur sont prises en compte en tant que paires de valeurs correspondantes servant à définir une propriété en pré-dialyse du sang d'un patient, dans laquelle le décalage dans le temps entre le premier et le deuxième moment se rapproche du temps d'écoulement du dialysat entre le premier et le deuxième capteur ou correspond à celui-ci.

2. Machine de dialyse selon la revendication 1, **caractérisée en ce que** le premier et le deuxième capteur sont des capteurs de conductivité, et que l'unité de calcul est réalisée de telle manière que les valeurs de mesure déterminées sont les conductivités du dialysat en amont et en aval du dialyseur, et que lesdites conductivités sont prise en compte en tant que paires de valeurs correspondantes servant à définir la concentration en ion en pré-dialyse, de préférence la concentration en ions de sodium en pré-dialyse dans le plasma sanguin du patient sous dialyse.

3. Machine de dialyse selon la revendication 1 ou 2, **caractérisée en ce que** l'unité de calcul est réalisée de telle manière que le décalage dans le temps est calculé à partir du volume du système hydraulique entre les deux capteurs et du débit de dialysat, ou que le décalage dans le temps est déterminé à l'aide de la différence dans le temps entre la détection d'une anomalie au niveau du premier et du deuxième capteur.

4. Machine de dialyse selon l'une quelconque des revendications 1 à 3, **caractérisée en ce que** l'unité de calcul est réalisée en outre de telle manière que le décalage dans le temps est adapté dans la mesure où la vitesse d'écoulement du dialysat se modifie durant le traitement.

5. Machine de dialyse selon l'une quelconque des revendications 1 à 4, **caractérisée en ce que** l'unité de calcul est réalisée en outre de telle manière qu'une conductivité équivalente de plasma en pré-dialyse est définie à l'aide des valeurs de conductivité déterminées en amont et en aval du dialyseur, et que la concentration en ions dans le plasma du patient sous dialyse est définie à partir de la conductivité équivalente de plasma en pré-dialyse par la suite.

6. Machine de dialyse selon la revendication 5, **caractérisée en ce que** l'unité de calcul est réalisée en outre de telle manière que la conductivité équivalente de plasma en pré-dialyse est définie par extrapolation de conductivités équivalentes de plasma instantanées, qui sont définies pour les plages d'évaluation dans le temps et/ou en tenant compte du décalage dans le temps à partir des valeurs de conductivité en amont et en aval du dialyseur, dans laquelle l'extrapolation comprend de préférence une régression des conductivités équivalentes de plasma instantanées en tant que fonction du temps ou en tant que fonction de la valeur Kt/V.
